# EUROPEAN PATENT APPLICATION

(11) **EP 4 197 524 A1**
(43) Date of publication of application: **21.06.2023**
(21) Application number: 21214973.6
(22) Date of filing: 16.12.2021
(51) Int. Cl.: A61K 9/00, A61P 31/12, A61P 31/14

(54) **INHALATION FORMULATIONS COMPRISING HEPARAN SULPHATE AS ACTIVE INGREDIENT**

(71) Applicant: CGM Research S.A.S. di Marco Rossi, 20121 Milano (IT)
(72) Inventor: RONCHI, Federica, 20100 Milano (IT); RONCHI, Celestino, 20100 Milano (IT)
(74) Representative: Bianchetti & Minoja with Trevisan & Cuonzo IPS SRL

(57) **Abstract**

The present invention relates to inhalation formulations comprising heparan sulphate as active ingredient and a suitable carrier, which are useful for the treatment and prevention of viral infections caused by Orthocoronavirinae.

## Description

The present invention relates to inhalation formulations comprising heparan sulphate as active ingredient and a suitable carrier, which are useful for the treatment and prevention of viral infections caused by Orthocoronavirinae.

### Prior art

Orthocoronavirinae is a subfamily of viruses, also known as coronaviruses, belonging to the Coronaviridae family, suborder Cornidovirineae, order Nidovirales.

It is divided into the genera Alphacoronavirus, Betacoronavirus, Gammacoronavirus and Deltacoronavirus, which include phylogenetically compact positive-sense RNA genogroups having a nucleocapsid with helical symmetry. Alphacoronaviruses and betacoronaviruses derive from the bat gene pool.

The genomic size of coronaviruses ranges between about 26 and 32 kilobases, which is exceptionally large for an RNA virus.

Their number is growing rapidly, and several new coronaviruses have been discovered recently, including SARS-CoV-1 in 2002, MERS-CoV in 2012 and SARS-CoV-2 in Wuhan (China) in 2019.

Coronaviruses are responsible for a number of diseases affecting mammals and birds.

In humans they cause respiratory tract infections, which are often mild, like the common cold, but in rare cases are potentially lethal, such as pneumonia and bronchitis.

Coronaviruses are responsible for serious epidemics of SARS in November 2002, MERS in 2012 and the new COVID-19 pandemic.

It is known that protein ACE2, a type I integral membrane protein abundantly expressed in the tissues lining the respiratory tract, is necessary for viral entry but is not the primary binding site on the cell surface. It has been demonstrated (Milewska A. et al., J Virol. 2014 Nov; 88(22): 13221-30. Epub 2014 Sep.3) that heparan sulphate proteoglycans act as adhesion molecules for coronavirus, increasing the density of the virus on the cell surface and possibly facilitating the interaction between HCoV and its receptor (ACE2).

Heparan sulphate (HS) is a sulphurylated glycosaminoglycan (GAG) present on the cell surface and in the extracellular matrix of multicellular organisms.

HS has an average molecular weight of 50 KDa and consists of disaccharide units of alpha-D-glucosamine bound via bond 1-4 to a unit of uronic acid.

Donalisio M. et al. (ASM Journals, Antimicrobial Agents and Chemotherapy Vol.56, No.10) highlight the interaction of respiratory syncytial virus (RSV) with heparan sulphate proteoglycans (HSPGs) on the cell surface to trigger infection. The virus-heparan sulphate interaction can therefore represent a target for the development of respiratory syncytial virus infection inhibitors. The study analysed a mini-library of linear, dimeric and dendrimeric peptides, containing clusters of basic amino acids, with a view to identifying peptides able to bind HSPGs, and therefore inhibit RSV adherence and infectivity.

Human metapneumovirus (HMPV) is a respiratory pathogen that causes acute diseases of the upper and lower respiratory tract. The mechanism whereby said virus recognises and accesses its target cell is still largely unknown. Chang A. et al. (ASM Journals - Journal of Virology, Vol.86, No.6) studied the initial stages of the bond with the virus and the consequent infection, and discovered that the first binding partner for HMPV is HS.

Clausen et al. (Cell 183, 1043-1057, 2020) have demonstrated that heparan sulphate is a cofactor which is also necessary for SARS-CoV-2 infection; heparan sulphate interacts with the domain of the bond to the SARSCoV-2 spike glycoprotein receptor, modifying its structure to facilitate its binding to ACE2. The authors suggest that HS mimetics, HS-degrading lyases and HS biosynthesis inhibitors could be used for therapeutic purposes.

EP 1 513 502 describes inhalation formulations comprising glycosaminoglycans as adjuvants for the release of therapeutic agents such as bronchodilators, steroids, antibacterials and antivirals.

WO 2021/194890 describes associations of heparin and N-acetylcysteine, administered by inhalation, which are useful for the treatment of respiratory infections caused by coronaviruses, optionally in combination with antivirals such as remdesivir, tocilizumab, lopinavir, sarilumab and beta interferon. None of the prior art documents therefore describe or postulate direct therapeutic applications of unmodified heparan sulphate as an agent able to treat or prevent viral infections.

### Description of the invention

It has now been discovered that heparan sulphate, when administered directly into the airways, effectively counteracts SARS-CoV2 infection by occupying as antagonist the binding site of the virus to the proteoglycans present on the cell membranes and extracellular matrices.

Heparan sulphate acts as an adherence molecule which occupies the binding site of the virus to the cells.

The object of the invention is therefore inhalation formulations comprising heparan sulphate (HS) as active ingredient, and a suitable carrier.

The HS-based inhalation preparations comprise powders which can be inhaled unmodified or dissolved in solutions, preferably saline solution or buffers, and then inhaled with an inhaler. Inhalation preparations containing HS can contain pharmaceutical grade excipients such as lactose, leucine, mannitol and magnesium stearate. The inhalation preparations preferably contain lactose.

The heparan sulphate dosage unit typically ranges between 10 and 50 mg. The carrier is preferably lactose monohydrate and the particle size distribution PSD 90 is less than 50 microns, preferably less than 10 microns.

The inhalation preparations containing HS are prepared by dissolving the active ingredient, together with the excipient, in water or organic solvent or in water/organic solvent mixtures in varying ratios, after which a powder with a PSD in the desired range is obtained by spray-drying technology.

Other excipients can be added to the HS/excipient mixture obtained by spray-drying, to improve the fluidity characteristics of the powder. The powder mixtures can then be used to fill hard gelatin and HPMC capsules and subsequently inhaled by means of conventional devices, such as an RS01 inhaler (Plastiape-Berry), having a body able to receive the capsule containing HS and pierce it with a perforation system to enable the contents of the capsule to be inhaled into the airways as a result of inspiration by the patient. Both pneumatic and ultrasound inhalers can be used.

The mixture of powders can also be packaged in vials, dissolved by adding a saline solution or isotonic buffer, and inhaled with an inhaler.

For the treatment and prevention of SARS CoV2 infections, the formulations according to the invention can be administered one to three times a day, depending on the clinical response, the severity of the disease and the patient's condition.

Detailed examples of formulations according to the invention are set out below.

### Example 1: capsules containing 10.0 mg of heparan sulphate

Qualitative/quantitative composition for an inhalation capsule containing 10.0 mg of HS
- Inhalation powder:
   ∘ HS 10.0 mg
   ∘ Respitose ML006 inhalation grade lactose monohydrate, DFE Pharma, 10.0 mg
- Capsugel Zephyr Vcaps^{®} Plus HPMC capsule, size no. 3.

### Preparation method for 1000 capsules of inhalation powder:

### Step #1 Preparation of solution:

In 200 ml of purified water, maintained under stirring with an IKA^{®}-WERKE Mod. RW16 basic stirrer at the speed of 200 rpm, add and dissolve in sequence:
∘ HS 10.0 g and after complete dissolution:
∘ 10.0 g of Respitose ML006 inhalation grade lactose monohydrate.

### Step #2 Obtaining inhalation powder by spray-drying

Atomise the solution obtained in step #1 with a BUCHI Mini Spray Dryer B-290 using the following operating parameters:
Input temperature: 150°C
Feed rate of solution: 20 ml/minute
Nitrogen flow: 0.5 bars.

### Step #3 Encapsulation of powder

Fill Capsugel Zephyr Vcaps^{®} Plus HPMC capsules, size no. 3, with the powder obtained in step #2, until a weight of 20.0 mg/capsule is reached.

### Step #4 Blister-packaging of capsules

Package the capsules in aluminum blister packs.

Heat-seal the capsules with a mechanical sealing machine.

### Step #5 Assembly and final packaging

A 10-capsule blister pack is packaged in a carton together with an inhaler device (RS01 Plastiape).

### Example 2: capsules containing 5.0 mg of heparan sulphate

Qualitative/quantitative composition for an inhalation capsule containing 5.0 mg of HS
- Inhalation powder:
   spray dry:
      ∘ HS 5.0 mg
      ∘ Respitose ML006 inhalation grade lactose monohydrate, DFE Pharma, 5.0 mg
   Final mixture:
      ∘ Respitose ML006 inhalation grade lactose monohydrate, DFE Pharma, 10.0 mg

### Preparation method for 1000 capsules of inhalation powder:

### Step #1 Preparation of solution

In 200 ml of purified water, maintained under stirring with an IKA^{®}-WERKE Mod. RW16 basic stirrer at the speed of 200 rpm, add and dissolve in sequence:
∘ HS 5.0 g
and after complete dissolution:
∘ 5.0 g of Respitose ML006 inhalation grade lactose monohydrate.

### Step #2 Obtaining inhalation powder by spray-drying technique

Atomise the solution obtained in step #1 with a BUCHI Mini Spray Dryer B-290 using the following operating parameters:
Input temperature: 150°C
Feed rate of solution: 20 ml/minute
Nitrogen flow: 0.5 bars.

### Step #3 Preparation of final mixture

10.0 g of the powder obtained by the spray-drying technique is recovered and combined with 10.0 g of lactose monohydrate.

Place the powders in a glass container and mix for 20 minutes with a TURBULA^{®} Schatz System Shaker Mixer Type T2C. Discharge the resulting mixture of powders and sieve through a 100 micron mesh sieve.

### Step #4: Encapsulation of powder

Fill Capsugel Zephyr Vcaps^{®} Plus HPMC capsules, size no. 3, with the powder obtained in step #3, until a weight of 20.0 mg/capsule is reached.

### Step #5 Blister-packaging of capsules

Package the capsules in aluminium blister packs.

Heat-seal the capsules with an O.M.A.R. s.r.l. Mod. Maxi Pack mechanical sealing machine.

### Step 6# Assembly and final packaging

A 10-capsule blister pack is then packaged in a carton together with an inhaler device (RS01 Plastiape).

### Example 3: vial containing 20.0 mg of heparan sulphate in saline solution

Qualitative/quantitative composition for a vial containing inhalation powder equivalent to 20.0 mg of HS

A 20 ml white glass vial contains:
Inhalation powder:
   ∘ HS 20.0 mg
   ∘ Respitose ML006 inhalation grade lactose monohydrate, DFE Pharma, 20.0 mg.
Solvent solution:
   ∘ Sodium chloride 180 mg
   ∘ Water for injectables q.s. to 20 ml.

### Preparation method for 1000 capsules of inhalation powder:

### Step #1 Preparation of HS solution

In 400 ml of purified water, maintained under stirring with an IKA^{®}-WERKE Mod. RW16 basic stirrer at the speed of 200 rpm, add and dissolve in sequence:
∘ HS 20.0 g
and after complete dissolution:
∘ 20.0 g of lactose monohydrate

### Step #2 Obtaining inhalation powder by spray-drying technique

Atomise the solution obtained in step #1 with a BUCHI Mini Spray Dryer B-290 using the following operating parameters:
Input temperature: 150°C
Feed rate of solution: 20 ml/minute
Nitrogen flow: 0.5 bars.

### Step #3 Vial filling

Fill type I white glass vials with a weight of 40 mg/vial of the powder obtained in step #2. Then seal with rubber cap and crimp cap.

### Preparation method for 1000 vials of solvent solution:

### Step #1 Preparation of solvent solution

Place 15 litres of water for injectables in a 20-litre stainless steel vessel and, maintaining it under stirring with an IKA^{®}-WERKE Mod. RW16 basic stirrer at the speed of 200 rpm, add and dissolve 180 g of sodium chloride.

When dissolution is complete, make up to a final volume of 20 litres.

### Step #2 Filtration of solution

Filter the resulting solution through Millipore filters with a porosity of 0.22 microns.

### Step #3 Vial filling

Fill vials with a volume of 20 ml/vial of saline solution filtered under sterile conditions. Then seal with rubber cap and crimp cap.

## Claims

1. Inhalation formulations comprising heparan sulphate as active ingredient, and a suitable carrier.

2. Formulations according to claim 1 in the form of inhalation powder.

3. Formulations according to claim 1 or 2 wherein the carrier is lactose monohydrate.

4. Formulations according to any one of claims 1 to 3 wherein the particle size distribution PSD 90 is less than 50 microns.

5. Formulations according to claim 4 wherein the particle size distribution PSD 90 is less than 10 microns.

6. Formulations according to any one of claims 1 to 3 comprising 10 to 50 mg of heparan sulphate per dosage unit.
